# EUROPEAN PATENT APPLICATION

(11) **EP 0 761 227 A1**
(43) Date of publication of application: **12.03.1997**
(21) Application number: 95920250.8
(22) Date of filing: 05.06.1995
(51) Int. Cl.: A61K 33/06, A61K 33/08, A61K 33/10, A61K 33/12, A61K 33/00

(54) **SOLID ANTACID AND PROCESS FOR PRODUCING THE SAME**

(30) Priority: 06.06.1994 JP 123862/94
(71) Applicant: YAMANOUCHI PHARMACEUTICAL CO. LTD., Tokyo 103 (JP)
(72) Inventor: SHIOZAWA, Hiroyoshi, Shizuoka 426 (JP); HASHIMOTO, Yoshimi, Shizuoka 421-01 (JP); TSUSHIMA, Keiko, Shizuoka 426-02 (JP); SETOGUCHI, Yoichi, Shizuoka 426 (JP)
(74) Representative: Geering, Keith Edwin
(86) International application number: JP9501094
(87) International publication number: WO9533469

(57) **Abstract**

A pharmaceutical composition useful for providing a solid antacid which has the effect of rapidly neutralizing acidity without unnecessarily enhancing the initial pH immediately after administration, is excellent in the effect of persistence of the optimum pH, and exhibits an excellent antacid effect based on the above effects. The composition contains as the active ingredient a combination of a lowly neutralizing antacid (one having an initial pH of less than 6 when tested by the modified Fuchs method using 30 ml of 0.05 N hydrochloric acid and a single dose of the sample, e.g. dry aluminum hydroxide gel) and a highly neutralizing antacid (one having a initial pH of 6 or above when tested similarly, e.g. magnesium hydroxide) coated with a pH-independent water-insoluble macromolecular base (e.g. aqueous ethylcellulose dispersion).

## Description

### Technical Field

The present invention relates to a pharmaceutical composition of a solid antacid which has excellent effects for promptly neutralizing gastric acids without excessively raising the initial pH inside the stomach immediately after the administration and for sustaining the pH inside the stomach at the optimum pH for a long period of time; and to a production process thereof.

### Background Art

Antacids have effects for neutralizing gastric acids or for protecting gastric mucosae and are used singly or in combination with other pharmaceuticals for the prevention and treatment of gastric hyperacidity, acute gastritis, chronic gastritis, peptic ulcer or the like or for the improvement of the symptoms accompanying such diseases. More specifically, antacids are used, as their direct actions, for neutralizing gastric acids, adjusting the pH to about 3-5 which is considered to be the optimum pH, and sustaining such effects for a certain period of time. At this optimum pH, activity of pepsin is suppressed so that its influence on the walls of the stomach can be much moderated. Rather, there is a potential danger of the secretion of gastric acids caused by rebound against the excessive rise in pH, particularly, excessive rise in the initial pH immediately after the administration (refer to, for example, *The Pharmacological Basis of Therapeutics*, ed. by Goodman and Gilman; 7th ed., 1209-1219, May 25, 1988; published by Hirokawa Shoten).

Accordingly, it is important that the antacids have excellent properties in:
(1) prompt neutralizing effects without an excessive rise in the initial pH immediately after administration, and
(2) effects of sustaining the optimum pH.

In the prior art, an invention relating to a chewable tablet is known, which comprises about 35-60 weight % of a water-insoluble antacid selected from the group consisting of aluminum hydroxide, magnesium hydroxide, magnesium oxide and magnesium silicate and mixtures thereof; 25-50 weight % of mannitol and about 4-10 weight % of urea, for providing a chewable tablet which can meet the demand for a solid antacid which is convenient and can be used easily compared with an internal liquid preparation, is chewable, can be swallowed without water, does not give unpleasant feeling at the time of chewing, and has sufficient antacid activity (refer to U.S. Patent No. 3,452,138). In the U.S. Patent, it is reported that an antacid which contains magnesium hydroxide and dried aluminum hydroxide gel and also contains mannitol and urea sustained the optimum pH of 3-5 for 81 minutes in an artificial gastric juice model.

In the above patent, however, there is neither a suggestion about the importance of prompt neutralization without an excessive rise in the initial pH immediately after the administration nor even a description about the initial pH. The duration specifically described above is not always sufficient when the patient's using convenience is taken into consideration, so the improvement is desired. It is said that use of urea, which is added to achieve the object of the invention in the above patent, should be avoided for ulcer or the like in consideration of its mucous stimulation. In the formulation indicated in the above patent, the amount of magnesium hydroxide, which is a cause for an excessive rise in the initial pH, is not more than the one-third of that of dried aluminum hydroxide gel, so that an excessive rise in the initial pH may not have occurred in this system. It is however necessary to increase the amount of magnesium hydroxide or the whole administration amount in order to prolong or improve the duration of the optimum pH. Accordingly, it is considered that the duration cannot be prolonged or improved further without an excessive increase in the initial pH, in view of the amount or mixing ratio of magnesium hydroxide described in the above patent.

An unexamined published Japanese patent application 6-56677 disclose an invention relating to "an antacid composition comprising a magnesium-bases antacid, and an organic acid or phosphoric acid or a salt thereof, which is to provide an antacid excellent in sustaining the gastric pH at about 3-5; and an unexamined published Japanese patent application 3-44319 discloses an invention relating to "an excessive release suppression type antacid which has an antacid substance uniformly dispersed and maintained in a polymer soluble in an acid range", which is to provide an antacid which does not cause excessive neutralization and has good sustainment. In either case, an excessive rise in the gastric pH can be suppressed, however, duration period of the initial pH at 3-5 is 30-60 minutes and is therefore insufficient.

An antacid having both of the above-described properties (1) and (2) has already been put on the market in the form of an internal liquid preparation, but that in the form of a solid preparation has not been known so far. There is accordingly a demand for the development of a solid preparation excellent in the above properties.

### Disclosure of the Invention

Various antacids have been known as those having effects for neutralizing gastric acids, thereby protecting gastric mucosae.

Representative examples of the antacid include sodium compound antacids such as sodium bicarbonate, aluminum compound antacids such as dried aluminum hydroxide gel and aluminum silicate, magnesium compound antacids such as magnesium oxide, magnesium hydroxide, magnesium carbonate and magnesium silicate, and aluminum-magnesium composite compound antacids such as magnesium aluminate, magnesium aluminosilicate hydrate, aluminium magnesium silicate, bismuth magnesium aluminosilicate hydrate and synthetic hydrotalcite, and calcium compound antacids such as calcium carbonate.

The above-exemplified antacids have characteristics as follows: The sodium compound antacids have fast acting properties but their reaction time is short. Sodium bicarbonate is known to react hydrochloric acid and emit carbon dioxide which becomes a stimulus and accelerates secretion of hydrochloric acid. Of the aluminum compound antacids, aluminum hydroxide is hardly soluble so that an unreacted portion precipitates and deposits on the surface of the walls of the stomach to some extent, thereby protecting the surface, and also it shows prolonged actions. The magnesium compound antacids are insoluble in water and generally stay in the stomach for a long time and has sustained effects. The aluminum-magnesium composite compound antacids have both of the characteristics of the aluminum compound antacids and magnesium compound antacids. The calcium compound antacids exhibit a relatively fast acting property and also sustainment (*Yakkyoku*, **40**(1), 325-329, 1989).

From the above antacids, the fast-acting one and sustained one are usually selected for use in combination. It is also possible to classify the antacids by the neutralizing capacity, that is, the antacid capacity to neutralize gastric acids. In such a case, an antacid having a high neutralizing capacity and that having a low neutralizing capacity are generally used in combination.

In antacids of any combination, however, a solid preparation produced by the conventionally known production process cannot have both of the above-described properties. That is, if an antacid having a high neutralizing capacity and that having a low neutralizing capacity are used in combination to increase the sustainment of the optimum pH, the antacid in which the initial pH after administration shows an excessive increase is obtained.

The term "the initial pH shows an excessive increase" as used herein means that the initial pH immediately after the administration rises too much and reaches the pH range where gastric acid is secreted by the reflex action for biophylaxis. It is difficult to clearly define the pH range which is the initial pH range immediately after the administration by which secretion of gastric juice is accelerated, because such a pH range differs with patients. In general, such a range means a range wherein the initial pH, that is, the pH within 10 minutes after the addition of a chemical is at least 6 when a test is conducted in accordance with the modified Fuchs method using an artificial gastric juice model which is regarded as a model clinically close to the conditions in the stomach. The term "modified Fuchs method" used herein means a testing method of antacid capacity using an artificial gastric juice model as described in Yamagata et al., *Kiso to rinsyo*, 24 (10), 1023-1028, 1990'' (the same will apply hereinafter).

In order to control the initial pH to the optimum pH, a method to reduce the amount of an antacid having a high neutralizing capacity (hereinafter called "high neutralizing capacity antacid") can be considered, but this method leads to the result that the sustained period of the optimum pH becomes short. Incidentally, the optimum pH of 3-5 is preferable in the present invention and the sustainment of the optimum pH means at least 3 hours when a test is conducted in 30 ml of 0.05N hydrochloric acid in accordance with the modified Fuchs method using an artificial gastric juice model.

Then, the present inventors have found that, when a test is conducted in accordance with the modified Fuchs method using an artificial gastric juice model, the optimum pH unexpectedly continues for a long time without excessively raising the initial pH by adding a high neutralizing capacity antacid at some interval after the addition of an antacid having a low neutralizing capacity (hereinafter called "low neutralizing capacity antacid"). Paying attention to the control of elution of the high neutralizing capacity antacid, the present inventors made an attempt to reproduce the above result by coating it with a polymer base. There are many kinds of polymer bases such as easily water-soluble polymers and water insoluble polymers, or according to another classification, pH dependent polymers such as gastric polymers and enteric polymers. The present inventors have found that, in order to achieve the above-described desired effects, it is necessary to select the polymer which is insoluble in water and dissolution thereof is not dependent on pH (hereinafter called "pH-independent and water-insoluble polymer bases" or called merely "polymer base"), from the above-described many polymers.

Based on such findings, the present invention has been completed. The present inventors have succeeded in providing an antacid with the above-described two characteristics by, in the combination of a high neutralizing capacity antacid and low neutralizing capacity antacid, coating the high neutralizing capacity antacid with a specific polymer base, thereby controlling the elution of the high neutralizing capacity antacid.

The present invention therefore relates to a pharmaceutical composition, which comprises, as antacid ingredients of a solid antacid, a low neutralizing capacity antacid, and a high neutralizing capacity antacid coated with a pH-independent and water-insoluble polymer base.

The present invention also relates to a production process of a pharmaceutical composition, which comprises spraying a solution or dispersion of a pH-independent and water-insoluble polymer base dissolved or dispersed in a solvent to a high neutralizing capacity antacid and drying to effect coating and then mixing the antacid so coated, a low neutralizing capacity antacid and necessary ingredients for preparation production to obtain a solid antacid.

The present invention is characterized in that the neutralization is conducted promptly without excessively raising the initial pH immediately after the administration and in addition, the optimum pH is sustained for long hours, which is achieved by coating a high neutralizing capacity antacid, which will otherwise cause an excessive increase in the initial pH immediately after administration, with a specific polymer base and then using it in combination with a low neutralizing capacity antacid.

In the above-described U.S. Patent and unexamined published Japanese patent application 6-56677, there is neither description nor suggestion about the suppression of an excessive rise in the initial pH, the control of the elution for the above suppression, coating of a high neutralizing capacity antacid in the case where a low neutralizing capacity antacid and the high neutralizing capacity antacid are used in combination, selection of a specific polymer base for the coating and attainment of sustainment of the optimum pH for at least 3 hours without excessively increasing the initial pH.

The invention disclosed in an unexamined published Japanese patent application 3-44319 in which an antacid substance is maintained in a dispersed form in an acidic water-soluble polymer is based on a completely different technical concept from that of the present invention which comprises a high neutralizing capacity antacid coated with a pH-independent and water-insoluble polymer base and a free low neutralizing capacity antacid.

The present invention will hereinafter be described in more detail.

The high neutralizing capacity antacid usable in the present invention is not particularly limited, as long as it is an antacid which has a high neutralizing capacity of gastric acids and achieve the objects of the present invention by coating and mixing, more specifically, as long as it is an antacid indicating an initial pH of at least 6 when a single dose as an antacid is added to 30 ml of 0.05N hydrochloric acid and a test is conducted in accordance with the modified Fuchs method. The term "single dose" herein means the one-third amount of the daily maximal amount described in *Iyakuhin Seizo Shisin* according to Japanese Welfare Ministry (edited by Nippon Koteisho Kyokai, published by Yakugyo Jiho-sha on September 25, 1992, 1992-edition) (the same will apply hereinafter). Particularly preferred examples include sodium compound antacids such as sodium bicarbonate, magnesium compound antacids such as magnesium oxide, magnesium hydroxide, magnesium carbonate and magnesium silicate, and calcium compound antacids such as calcium carbonate. More preferred are magnesium compound antacids. Of these, magnesium hydroxide is particularly preferred, because it has been used as an internal liquid preparation for oral administration for 20 years or more and its safety has been confirmed.

The low neutralizing capacity antacid is not particularly limited as long as it is an antacid having a low neutralizing capacity and can achieve the desired object of the present invention by mixing with the coated high neutralizing capacity antacid, more specifically, as long as it is an antacid which has an initial pH less than 6 when a single dose is added as an antacid to 30 ml of 0.05N hydrochloric acid, that is, the one-third of the daily maximal amount described in the above *Iyakuhin Seizo Shisin* and test is conducted in accordance with the modified Fuchs method. Particularly preferred examples include aluminum-magnesium composite compound antacids such as magnesium aluminate, dimagnesium silicate aluminate, magnesium metasilicate aluminate, magnesium bismuth silicate aluminate and synthetic hydrotalcite; and aluminum compound antacids such as dried aluminum hydroxide gel and aluminum silicate. More preferred are aluminum compound antacids. Of these, dried aluminum hydroxide gel is particularly preferred, because it has been used as an internal liquid preparation for oral administration for 20 years or more and its safety has been confirmed.

As the high neutralizing capacity antacid or low neutralizing capacity antacid, two or more antacids can be used in combination, respectively.

The combination of the high neutralizing capacity antacid and the low neutralizing capacity antacid is not particularly limited as long as it is the combination of the antacids exemplified above in the respective descriptions. Preferred is a combination of a magnesium compound antacid and an aluminum compound antacid, with a combination of magnesium hydroxide and dried aluminum hydroxide gel being more preferred.

Concerning the amounts of the low neutralizing capacity antacid and high neutralizing capacity antacid coated with a pH-independent and water-insoluble polymer base, any amount is possible if the excellent properties are achieved in (1) prompt neutralizing effects without excessively increasing the initial pH after administration and (2) sustained effects of the optimum pH.

In particular, the amounts of the low neutralizing capacity antacid and the coated high neutralizing capacity antacid may differ depending on various conditions such as kind of the antacid, kind and amount of the polymer base used for coating, addition or non-addition of uncoated high neutralizing capacity antacid and the like and therefore cannot be determined generally. However, in order to achieve the objects of the present invention, it is preferred that the amount of low neutralizing capacity antacid *per se* and the amount of high neutralizing capacity antacid *per se* are at a ratio of 0.2 - 2:1.

The amounts of these antacids are described in further detail using, as an example, an antacid which comprises magnesium hydroxide as the high neutralizing capacity antacid and dried aluminum hydroxide gel as the low neutralizing capacity antacid, which was a particularly preferred embodiment.

Incidentally, dried aluminum hydroxide gel contains at least 50.0% of aluminum oxide as listed in the *Japanese Pharmacopoeia* XII. The purity of dried aluminum hydroxide gel differs with the lot of the raw material so that the description will hereinafter be made concerning magnesium oxide.

Concerning the amounts (doses) of magnesium hydroxide and dried aluminum hydroxide gel, the amounts which have been confirmed to be safe based on the results of their use for 20 years or more as an internal liquid preparation for oral administration, that is, about 400 mg for magnesium hydroxide and about 225 mg as aluminum oxide are most preferred. These amounts can be increased or decreased optionally according to the symptoms of the patients or the like. When the amount of magnesium hydroxide and the amount in terms of aluminum oxide are 400 mg and 225 mg, respectively, the amount of magnesium hydroxide which is a high neutralizing capacity antacid to be coated with a pH-independent and water-insoluble polymer base is about 300-400 mg. This is because, if the amount of magnesium hydroxide uncoated with the polymer base exceeds 100 mg, an excessive increase in the initial pH is observed (refer to Test Example 2).

Concerning the mixing ratio, it is particularly preferred to mix magnesium hydroxide and aluminum oxide at a ratio of about 1:0.56 because of the same reasons as described above. This ratio can also be increased or decreased optionally according to the symptoms of the patient or the like. In this combination, it is preferred that the amount of magnesium hydroxide to be coated with the polymer base is at least 75%.

The pH-independent and water-insoluble polymer base usable in the present invention is not particularly limited as long as the polymer base is pharmaceutically acceptable in general, pH-independent and water-insoluble polymer base which can achieve the object of the present invention. Examples thereof include ethyl cellulose, aqueous dispersion-type ethyl cellulose (e.g., Aquacoat (product name), a product of FMC Inc.), a Polyethylacrylate, methylmethacrylate, trimethylammonioethylmethacrylatechlorid (Eudragit RS 100, RS30D (product name), products of Röhn Pharma) and an emulsion of an ethyl acrylate-methyl methacrylate copolymer (e.g., Eudragit NE30D (a product name), a product of Röhn Pharma). These polymers can be used either singly or in combination. Particularly preferred is a polymer base containing aqueous dispersion-type ethyl cellulose and an emulsion of an ethyl acrylate-methyl methacrylate copolymer in combination. The coating solution containing the polymer base can optionally contain a plasticizer, lubricant, defoaming agent, colorant, surfactant and the like.

The solvent usable in the present invention is not particularly limited as long as the above-described pharmaceutically acceptable polymer base can be dissolved or dispersed in it. Examples of the solvent include water and organic solvents such as methanol, ethanol, isopropanol, methylene chloride, hexane and acetone. These solvents can be used either singly or in combination.

The amount (ratio) of the polymer base to be dissolved or dispersed in a solvent cannot be generally defined because it differs with the combination of the solvent and polymer base. However, it is usually about 30 weight % or smaller.

The coating amount of the solution or dispersion of the polymer base dissolved or dispersed in a solvent for coating the high neutralizing capacity antacid cannot be generally defined because it depends on the combination of the solvent and the polymer base and the form to be coated, particularly, the particle size. The amount is about 5-20 weight % when the particle size is as large as that of a granule and 20-100 weight % when the particle size is smaller than the above.

Although the high neutralizing capacity antacid is coated with the polymer base, gaps among many layers of the coated product or strains of the molecular chain of the polymer base which has coated the antacid become a water entering path or antacid outlet path, from which the coated antacid is released.

The high neutralizing capacity antacid coated with the polymer base is mixed with the low neutralizing capacity antacid, which makes it possible to have the above-described two properties. A solid antacid can be obtained by mixing the high neutralizing capacity antacid coated with the polymer base and uncoated low neutralizing capacity antacid with further necessary ingredients for the preparation production. That is, these antacids, together with an excipient or a compound having an activity as a pharmaceutical (and, if necessary, with a disintegrator, binder, lubricant, fluidizing agent, perfume and colorant, stabilizer, coating agent, etc.) can be formed into tablets, chewable tablets, granules, powders, fine granules, pills or capsules in a conventional manner.

Examples of the excipient for use in the present invention include mannitol, lactose, starch, xylitol, erythritol and sorbitol.

For example, a low neutralizing capacity antacid (such as dried aluminum hydroxide gel) and an excipient such as mannitol, lactose or starch and, if necessary, other active ingredients and additives are granulated in a fluidized bed granulator by using a binder such as hydroxypropylmethylcellulose, and a high neutralizing capacity antacid (such as magnesium hydroxide) coated with a polymer base is mixed with the resulting granulated mixture, whereby granules, fine granules or powders can be obtained. After adding a lubricant, the resulting mixture is tabletted by a rotary tabletting machine to obtain tablets or chewable tablets. Alternatively, the mixture is filled in capsule containers in a conventional manner, whereby capsules can be obtained.

At this time, in order to adjust the initial pH within an extent not excessively increasing the initial pH immediately after the administration and also to adjust the sustainment of the optimum pH, a high neutralizing capacity antacid not subjected to a coating treatment with a polymer base can be added to the coated one or can be replaced partially with the coated one.

The pharmaceutical composition according to the present invention can be blended with other compounds having activity as pharmaceuticals. Examples thereof include histamine H₂ receptor antagonist (H₂ blocker); proton pump inhibitors such as omeprazole and lansoprazole; those having antibacterial activity against *Helicobacter pylori* such as amoxicillin, minocycline, erythromycin and ofloxacin; enterokinesis regulator such as trimebutine maleate, cisapride and domperidone; prostaglandin; sucralfate; gefarnate; cetraxate; and teprenone.

Further examples include stomachic crude ingredients such as swertia herb, cinammon bark, l-menthol and dl-menthol; gastroenteric function regulators such as carnitine chloride and bethanechol chloride; stomachics such as dry yeast, amino acid agents (e.g., aminoacetic acid and dihydroxyaluminum amino acetate); gastric acid secretion inhibitors such as scopolia extract, digestive enzymes such as starch digesting enzyme, protein digesting enzyme, lipid digesting enzyme and cellulose digesting enzyme; digestants such as cholagogues (e.g., ursodesoxycholic acid, oxycholanoates, cholic acid, bile powder, bile extract (powder), dehydrocholic acid and animal bile (including bear bile); intestine regulating agents such as viable microorganism ingredients; adsorbents such as kaolin, natural aluminum silicate and aluminum hydroxynaphthoate; antidiarrheals drugs such as coating agents (e.g., precipitated calcium carbonate and calcium lactate; mucosa repairing agents such as sodium azulenesulfonate, aldioxa, glycyrrhizic acid, salts thereof, licorice extract, L-glutamine, potassium copper chlorophillin, histidine chloride, porcine stomach wall pepsin degraded product, methylmethioninesulfonium chloride, malloti cortex and corydalis tuber; and defoaming agents such as dimethylpolysiloxane.

Then, the production methods will be described in detail. The present invention should not be construed as being limited thereto.

The coating may be conducted using a method generally employed. A high neutralizing capacity antacid (e.g., magnesium hydroxide) alone or with an excipient such as lactose is kneaded in a stirring granulator, using an aqueous solution of hydroxypropylcellulose as a binder. The kneaded mass is pulverized and dried, followed by sieving (for example, 24-mesh sieve) to give granules. The granules so obtained are charged in a fluidized bed coating machine, where a coating solution is sprayed and coating is conducted until the coating amount reaches the desired extent.

As an another method, magnesium hydroxide, crystalline cellulose and polyvinyl pyrrolidone are charged in a high-speed stirring granulator, purified water is added thereto and granulation is conducted until granules take a spherical shape, followed by drying. The granules so obtained are charged in a fluidized bed coating machine, where a coating solution is sprayed and coating is effected until the coating amount reaches the desired extent.

As still another method, magnesium hydroxide, lactose and starch are charged in a stirring granulator and the resulting mixture is kneaded using an aqueous solution of polyvinyl pyrrolidone as a binder. The kneaded mass so obtained is charged in an extrusion granulator to form cylindrical granules, followed by subjecting rounding treatment in a centrifugal fluid granulator and drying. The granules so obtained are coated as described above in a centrifugal fluid granulator.

Other production methods include a method in which a paste obtained by dispersing magnesium hydroxide in a binder solution is dropped by an atomizer using a spray drier to obtain granules in a spherical form and then the granules so obtained are coated and a method in which magnesium hydroxide alone or with an excipient is granulated with a coating solution by using a fluidized bed granulator, followed by further coating.

As a still further method, crystalline cellulose granules or sucrose granules as a nucleus are coated with powder or dispersion of magnesium hydroxide together with a binder solution in a centrifugal fluid granulator, followed by coating the granules so obtained.

There are thus various coating methods, and the optimum method may be selected in consideration of the working efficiency and cost and the present invention is not limited by these methods. The pharmaceutical composition according to the present invention having the above-described two properties can be obtained by mixing the coated product obtained by one of the above-described coating methods and a low neutralizing capacity antacid in the above-described conventional manner.

### Brief Description of the Drawings

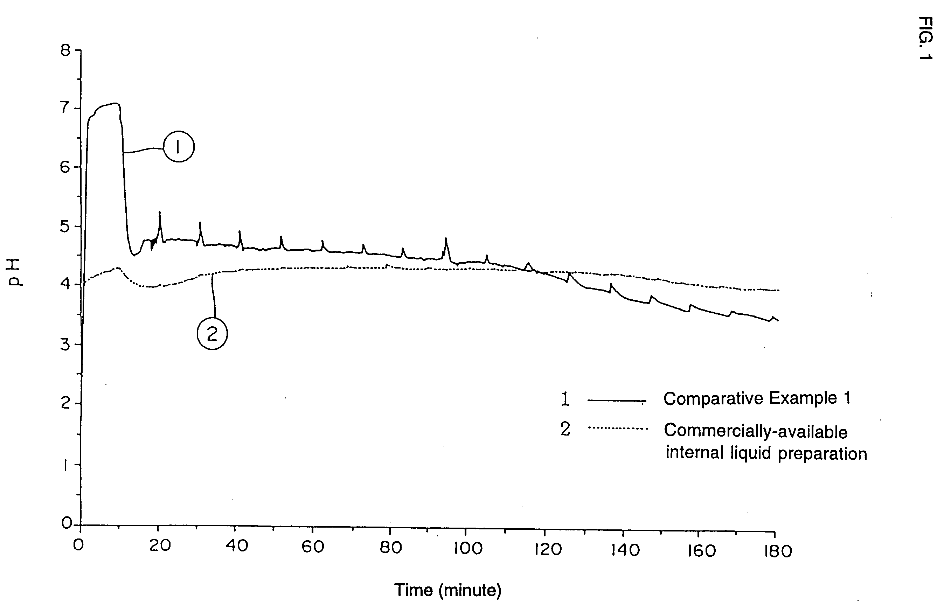

FIG. 1 illustrates results of the antacid capacity sustainment test on the chewable tablet obtained in Comparative Example 1 and a commercially-available internal liquid preparation having the same composition, in accordance with the modified Fuchs method using an artificial gastric juice model.

FIG. 2 illustrates results of the antacid capacity sustainment test on the coated product obtained in Example 1-(a), in accordance with the modified Fuchs method using an artificial gastric juice model.

FIG. 3 illustrates results of the antacid capacity sustainment test on the chewable tablets obtained in Example 2-(b) and Example 4-(b), in accordance with the modified Fuchs method using an artificial gastric juice model.

### Best Modes for Carrying Out the Invention

The present invention will hereinafter be described more specifically by the examples of the pharmaceutical composition according to the present invention. However, the present invention should not be construed as being limited to these Examples.

### Example 1

(a) In a fluidized bed granulator FLO-1 (manufactured by Fleund Inc.; the same will apply hereinafter), 500 parts of magnesium hydroxide were charged. After the granulation, coating was conducted under the conditions of blast temperature of 50°C, spray air pressure of 2.0 kg/cm² and a flow rate of 9 g/min, using a solution of 150 parts of ethyl cellulose dissolved in 1,350 parts of ethanol as a coating solution to give 10%-, 15%- and 20%-coated magnesium hydroxide as solids.
(b) A mixture obtained by mixing 137.3 parts of dried aluminum hydroxide gel and 180 parts of mannitol was sieved through a 28-mesh sieve and charged in the fluidized bed granulator FLO-1 together with 49.5 parts of lactose and 45.6 parts of starch, followed by granulation using 177.3 parts of a 7.5% aqueous solution of hydroxypropylcellulose (hereinafter abbreviated as "HPC"). Then, 354.7 parts of the resulting granulated mixture, 133.3 parts of the 20%-coated product obtained above in Example 1-(a), 0.5 part of a flavor, 1.5 parts of light anhydrous silicic acid and 10 parts of magnesium stearate were mixed in a mixer for 15 minutes. The resulting mixture was pressed into 1.8 g tablets by a punch of 18 mm in diameter on a rotary tabletting machine to give chewable tablets containing, per tablet, 400 mg of magnesium hydroxide and 412 mg of dried aluminum hydroxide gel (225 mg in terms of aluminum oxide).

### Example 2

(a) In a fluidized bed granulator FLO-1, 400 parts of magnesium hydroxide were charged. After the granulation, coating was conducted under the conditions of blast temperature of 90°C, spray air pressure of 1.5 kg/cm² and a flow rate of 8 g/min, using a coating solution composed of 1,000 parts of an aqueous dispersion of ethyl cellulose (Aquacoat (product name), a product of FMC Corp.) and 75 parts of triethyl citrate to give 40%- and 60%-coated magnesium hydroxide as solids.
(b) In a manner similar to Example 1-(b) except that the 40%-coated product obtained in Example 2-(a) was used, a chewable tablet containing, per tablet, 400 mg of magnesium hydroxide and 412 mg of dried aluminum hydroxide gel (225 mg in terms of aluminum oxide) was obtained. The increase in weight caused by coating was adjusted by reducing the amounts of mannitol and lactose.

### Example 3

(a) In a manner similar to Example 2-(a) except that a mixed solution of 560.1 parts of an emulsion of a Polyethylacrylate, methylmethacrylate, trimethylammonioethylmethacrylatechlorid (Eudragit NE30D (product name), a product of Röhn Pharma Inc.; the same will apply hereinafter) and 240 parts of an aqueous dispersion of ethyl cellulose was used as the coating solution, coating was conducted to give 40%- and 60%-coated magnesium hydroxide as solids.
(b) A mixture obtained by mixing 137.3 parts of dried aluminum hydroxide gel and 141.7 parts of mannitol was sieved through a 28-mesh sieve, followed by charging in a fluidized bed granulator FLO-1 together with 49.5 parts of lactose and 45.6 parts of starch. They were granulated using 177.3 parts of a 7.5% aqueous solution of HPC. Then, 322.8 parts of the resulting granulated mixture, 144.4 parts of the 60%-coated product obtained above in Example 2-(b) and 20.8 g of untreated magnesium hydroxide, and also 0.5 part of a flavor, 1.5 parts of light anhydrous silicic acid and 10 parts of magnesium stearate were mixed in a mixer for 15 minutes. The resulting mixture was pressed into 1.8 g tablets by a punch of 18 mm in diameter on a rotary tabletting machine to give chewable tablets containing, per tablet, 520 mg of 60%-coated product (325 mg in terms of magnesium hydroxide), 75 mg of untreated magnesium hydroxide and 412 mg of dried aluminum hydroxide gel (225 mg in terms of aluminum oxide).

### Example 4

(a) In a manner similar to Example 2-(a) except that a mixed solution of 560.1 parts of an aqueous dispersion of ethyl cellulose and 240 parts of an emulsion of an ethyl acrylate-methyl methacrylate copolymer was used as a coating solution, coating was conducted to give 40%- and 60%-coated magnesium hydroxide as solids.
(b) In a manner similar to Example 1-(b) except that the 60%-coated product obtained in Example 4-(a) was used, chewable tablets containing, per tablet, 400 mg of magnesium hydroxide and 412 mg of dried aluminum hydroxide (225 mg in terms of aluminum oxide) were obtained. The increase in weight caused by coating was adjusted by reducing the amounts of mannitol and lactose.

### Example 5

A mixture obtained by mixing 137.3 parts of dried aluminum hydroxide gel and 136.6 parts of mannitol was sieved through a 28-mesh sieve, followed by charging in a fluidized bed granulator FLO-1 together with 49.5 parts of lactose and 45.6 parts of starch. They were then granulated using 177.3 parts of a 7.5% aqueous solution of HPC. In a mixer, 318.5 parts of the granulated mixture so obtained, 155.6 parts of the 60%-coated product obtained in Example 2-(a) and 13.9 parts of untreated magnesium hydroxide, and also 0.5 part of a flavor, 1.5 parts of light anhydrous silicic acid and 10 parts of magnesium stearate were mixed for 15 minutes. The resulting mixture was pressed into 1.8 g tablets by a punch of 18 mm in diameter on a rotary tabletting machine to give chewable tablets containing, per tablet, 560 mg of 60%-coated product (350 mg in terms of magnesium hydroxide), 50 mg of untreated magnesium hydroxide and 412 mg of dried aluminum hydroxide gel (225 mg in terms of aluminum oxide).

### Example 6

A mixture obtained by mixing 175.9 parts of dried aluminum hydroxide gel, 173.7 parts of lactose and 43.4 parts of starch were mixed was sieved through a 28-mesh sieve, followed by charging in a fluidized bed granulator FLO-1. They were then granulated using 100 parts of a 7.0% aqueous solution of HPC. The granulated mixture was sieved through a 32-mesh sieve. Then, 312.3 parts of the sieved mixture were mixed with 186.7 parts of the 40%-coated product obtained in Example 3-(a) and sieved through a 32-mesh sieve and 1.0 part of light anhydrous silicic acid in a mixer for 15 minutes. As a result, powders containing, per 1.5 g, 400 mg of magnesium hydroxide and 412 mg of dried aluminum hydroxide gel (225 mg in terms of aluminum oxide) were obtained.

### Example 7

In a stirring granulator, 400 parts of magnesium hydroxide, 150 parts of lactose and 50 parts of starch were charged, followed by the addition of 120 parts of a 15% aqueous solution of HPC. The resulting mixture was then kneaded. The kneaded mixture was granulated in an extrusion granulator having a bore size of 0.8 mm, followed by rounding treatment by a centrifugal fluid granulator and then drying to give cylindrical granules. In a fluidized bed granulator FLO-1, 500 parts of the granules so prepared were charged and were subjected to 7% coating as solids using a solution of 30 parts of ethyl cellulose dissolved in 270 parts of ethanol as a coating solution.

Separately, 412 parts of dried aluminum hydroxide gel, 281.9 parts of lactose and 120.8 parts of starch were charged in a stirring granulator. They were kneaded using 160 parts of a 15% aqueous solution of HPC, followed by granulating in an extrusion granulator, whereby cylindrical granules were formed. In a mixer, 132.3 parts of the magnesium hydroxide-containing granules and 166.7 parts of the dry aluminum hydroxide-containing granules were mixed for 10 minutes to give granules containing, per 1.5 g, 400 mg of magnesium hydroxide and 412 mg of dried aluminum hydroxide gel (225 mg in terms of aluminum oxide).

### Example 8

In a fluidized bed granulator FLO-1, 500 parts of magnesium hydroxide were charged, followed by granulating using as a coating solution a solution of 150 parts of ethyl cellulose dissolved in 1,350 parts of ethanol under the conditions of a blast temperature of 50°C, spray air pressure of 2.0 kg/cm² and a flow rate of 9 g/min to give 20%-coated magnesium hydroxide was obtained as a solid. Separately, 500 parts of dried aluminum hydroxide gel were charged in a fluidized bed granulator FLO-1 and granulated using 150 parts of a 10% aqueous solution of HPC as a binder. Then, 480 parts of the coated magnesium hydroxide, 424.4 parts of dried aluminum hydroxide gel so granulated and 4.5 parts of magnesium stearate were mixed. The resulting mixture (303 mg) was filled in No.1 capsules by a Hebriger capsule filling machine to give capsules containing, per capsule, 133.3 mg of magnesium hydroxide and 137.3 mg of dried aluminum hydroxide gel.

### Example 9

In a fluidized bed granulator FLO-1, 400 parts of dried aluminum hydroxide gel were charged, followed by granulation using 124.5 parts of a 10% aqueous solution of HPC as a binder. In a mixer, 86.6 parts of the granules so obtained, 130.6 parts of the 60%-coated product obtained in Example 4-(a), 63.3 parts of crystalline cellulose, 15 parts of partially alphanized starch, 3 parts of talc and 1.5 parts of magnesium stearate were mixed for 10 minutes. The resulting mixture was pressed into 0.49 g tablets by a punch of 16 mm in longer diameter and 7 mm in shorter diameter on a tabletting machine to give an ovally-shaped tablet containing, per tablet, 133.3 mg of magnesium hydroxide and 137.7 mg of dried aluminum hydroxide gel.

### Example 10

In a manner similar to Example 2-(a) except that a mixed solution of 240 parts of an aqueous dispersion of ethyl cellulose, 160 parts of an emulsion of a Polyethylacrylate, methylmethacrylate, trimethylammonioethylmethacrylatechlorid and 200 parts of purified water was used as the coating solution, the coating was conducted to give 30%-coated magnesium hydroxide as a solid.

### Example 11

In a manner similar to Example 2-(a) except that a mixed solution of 200 parts of an aqueous dispersion of ethyl cellulose, 200 parts of an emulsion of a Polyethylacrylate, methylmethacrylate, trimethylammonioethylmethacrylatechlorid and 200 parts of purified water was used as the coating solution, the coating was conducted to give 30%-coated magnesium hydroxide as a solid.

### Example 12

In fluidized bed granulator FLO-1, 500 parts of magnesium hydroxide were charged and they were granulated using a solution of 37.5 parts of polyvinyl pyrrolidone in 112.5 parts of purified water as a binder. In a manner similar to Example 2-(A), coating of the granulated product so obtained was conducted using as a coating solution a mixed solution of 270 parts of an aqueous dispersion of ethyl cellulose and 180 parts of an emulsion of a Polyethylacrylate, methylmethacrylate, trimethylammonioethylmethacrylatechlorid to give 25%-coated magnesium hydroxide granules as a solid.

### Example 13

In a manner similar to Example 12 except that a mixed solution of 315 parts of an aqueous dispersion of ethyl cellulose and 135 parts of an emulsion of a Polyethylacrylate, methylmethacrylate, trimethylammonioethylmethacrylatechlorid was used as the coating solution, the coating was conducted to give 25%-coated magnesium hydroxide granules as a solid.

### Example 14

In a manner similar to Example 12 except that a mixed solution of 180 parts of an emulsion of a Polyethylacrylate, methylmethacrylate, trimethylammonioethylmethacrylatechlorid, 180 parts of an aqueous dispersion of ethyl cellulose and 360 parts of purified water was used as the coating solution, the coating was conducted to give 20%-coated magnesium hydroxide granules as a solid.

### Example 15

After 265.85 parts of dried aluminum hydroxide gel and 468.0 parts of xylitol powder were mixed, the resulting mixture was sieved through a 28-mesh sieve, charged in a fluidized bed granulator FLO-1 and then granulated using 386.53 parts of a 7.5% aqueous solution of HPC. In a mixer, 704.16 parts of the granulated product, 322.8 parts of the 25%-coated product obtained in Example 12, 1.08 parts of a flavor, 3.24 parts of light anhydrous silicic acid and 21.6 parts of magnesium stearate were mixed for 15 minutes to give a powdery mixture for tablets.

### Example 16

The powdery mixture for tablets (500 parts) obtained in Example 15 was taken and pressed into 1.755 g tablets by a punch of 18 mm in diameter on a rotary tabletting machine to give chewable tablets containing, per tablet, 400 mg of magnesium hydroxide and 409 mg of dried aluminum hydroxide gel (225 mg in terms of aluminum oxide).

### Example 17

The powdery mixture for tablets (500 parts) obtained in Example 15 was taken and pressed into 0.8774 g tablets by a punch of 15 mm in diameter on a rotary tabletting machine to give chewable tablets containing, per tablet, 200 mg of magnesium hydroxide and 204.5 mg of dried aluminum hydroxide gel (112.5 mg in terms of aluminum oxide) and whose single dose was two tablets a time.

### Example 18

After 224.95 parts of dried aluminum hydroxide gel and 396.0 parts of pulverized erythritol were mixed, the resulting mixture was sieved through a 28-mesh sieve, charged in a fluidized bed granulator FLO-1, and then granulated using 327.1 parts of a 7.5% aqueous solution of HPC. In a mixer, 586.8 parts of the resulting granulated product, 269.0 parts of the 25%-coated product obtained in Example 12, 0.9 part of a flavor, 2.7 parts of light anhydrous silicic acid and 18.0 parts of magnesium stearate were mixed for 15 minutes to give a powdery mixture for tablets.

### Example 19

The powdery mixture for tablets (400 g) obtained in Example 18 was taken and pressed into 1.755 g tablets by a punch of 18 mm in diameter on a rotary tabletting machine to give chewable tablets containing, per tablet, 400 mg of magnesium hydroxide and 409 mg of dried aluminum hydroxide (225 mg in terms of aluminum oxide).

### Example 20

The powdery mixture for tablets (400 g) obtained in Example 18 was taken and pressed into 0.8774 g tablets by a punch of 15 mm in diameter on a rotary tabletting machine to give chewable tablets containing, per tablet, 200 mg of magnesium hydroxide and 204.5 mg of dried aluminum hydroxide (112.5 mg in terms of aluminum oxide) and whose single dose was two tablets a time.

### Example 21

After 224.95 parts of dried aluminum hydroxide gel, 198.0 parts of pulverized erythritol and 198.0 parts of xylitol powder were mixed, the resulting mixture was sieved through a 28-mesh sieve, charged in a fluidized bed granulator FLO-1 and then granulated using 327.1 parts of a 7.5% aqueous solution of HPC. Then, 586.8 parts of the resulting granulated product, 269.0 parts of the 25% coated product obtained in Example 13, 0.9 part of a flavor, 2.7 parts of light anhydrous silicic acid and 18.0 parts of magnesium stearate were mixed in a mixer for 15 minutes. The resulting mixture was pressed into 1.755 g tablets by a punch of 18 mm in diameter on a rotary tabletting machine to give chewable tablets containing, per tablet, 400 mg of magnesium hydroxide and 409 mg of dried aluminum hydroxide gel (225 mg in terms of aluminum oxide).

### Example 22

After 224.95 parts of dried aluminum hydroxide gel, 198.0 parts of pulverized erythritol and 198.0 parts of sorbitol powder were mixed, the resulting mixture was sieved through a 28-mesh sieve, charged in a fluidized bed granulator FLO-1 and then pulverized using 327.1 parts of a 7.5% aqueous solution of HPC. In a mixer, 586.8 parts of the resulting granulated mixture, 269.0 parts of the 25%-coated product obtained in Example 13, 0.9 part of a flavor, 2.7 parts of light anhydrous silicic acid and 18.0 parts of magnesium stearate were mixed for 15 minutes. The resulting mixture was pressed into 1.755 g tablets by a punch of 18 mm in diameter on a rotary tabletting machine to give chewable tablets containing, per tablet, 400 mg of magnesium hydroxide and 409 mg of dried aluminum hydroxide gel (225 mg in terms of aluminum oxide).

### Industry Applicability

The pharmaceutical composition according to the present invention is excellent in the effects of promptly neutralizing the initial pH immediately after administration without excessively raising it and also excellent in the effects of sustaining the optimum pH, so that it is useful as a composition capable of providing a solid antacid which exhibits good antacid effects based on the above effects.

The excellent antacid effects of the pharmaceutical composition according to the present invention were confirmed by the test which will be described below. Incidentally, a preparation example of a chewable tablet comprising an uncoated high neutralizing capacity antacid is shown in Comparative Example as a control.

### Comparative Example 1

In a fluidized bed granulator FLO-1, 100 parts of magnesium hydroxide, 103 parts of dried aluminum hydroxide gel, 120 parts of mannitol, 50 parts of lactose and 47.3 parts of starch were charged, followed by granulation using 180 parts of a 7.5% aqueous solution of HPC as a binder to give granules. To the granules so obtained, 2.25 parts of light anhydrous silicic acid, 0.45 part of a flavor and 13.5 parts of magnesium stearate were added and mixed. The resulting mixture was pressed into 1.8 g tablets by a punch of 18 mm in diameter on a rotary tabletting machine to give chewable tablets containing, per tablet, 400 mg of magnesium hydroxide and 412 mg of dried aluminum hydroxide gel (225 mg in terms of aluminum oxide).

### Test Example 1

With respect to the chewable tablets obtained in Comparative Example 1 and a commercially-available internal liquid preparation having the same composition, a sustainment test of antacid capacity was conducted in an artificial gastric juice model. To 30 ml of 0.05N hydrochloric acid under stirring at 37°C and 300 rpm, one tablet in the case of the chewable tablet and a single dose in the case of the internal liquid preparation were added, respectively. From ten minutes after the addition, 0.05N hydrochloric acid was poured into each of the resulting mixtures at a rate of 2 ml/min and the pH of the test solution was recorded continuously. Incidentally, the tablet was provided for the test after crude pulverization. According to the test results, it has been found that the chewable tablet which was an internal solid preparation indicated a high initial pH and exceeded the optimum pH.

### Test Example 2

To 412 mg of dried aluminum hydroxide gel, magnesium hydroxide was added in various amounts and as in Test Example 1, the sustainment test of antacid capacity was conducted. Incidentally, the test was conducted for a period in which it was confirmed that the amount of a high neutralizing capacity-having antacid does not cause an excessive rise in the initial pH. The duration of the optimum pH was confirmed, concerning the amounts of 75 mg and 400 mg. As a result, it was confirmed that, when magnesium hydroxide was added in an amount of 100 mg or smaller, the initial pH could be suppressed. However, the duration of the-optimum pH was short.

**Table 1**

| | Amount of magnesium hydroxide added | | | | | |
|---|---|---|---|---|---|---|
| | 400 mg | 200 mg | 150 mg | 100 mg | 75 mg | 50 mg |
| Initial pH | 1.32 | 1.43 | 1.44 | 1.43 | 1.38 | 1.42 |
| pH after 10 min. | 7.77 | 7.34 | 6.96 | 5.85 | 5.36 | 4.91 |
| pH after 20 min. | 4.75 | / | / | / | 3.67 | / |
| pH after 30 min. | 4.58 | / | / | / | 3.71 | / |
| pH after 40 min. | 4.49 | / | / | / | 3.68 | / |
| pH after 60 min. | 4.36 | / | / | / | 3.56 | / |
| pH after 80 min. | 4.27 | / | / | / | 2.32 | / |
| pH after 120 min. | 4.14 | / | / | / | / | / |
| pH after 180 min. | 4.02 | / | / | / | / | / |

### Test Example 3

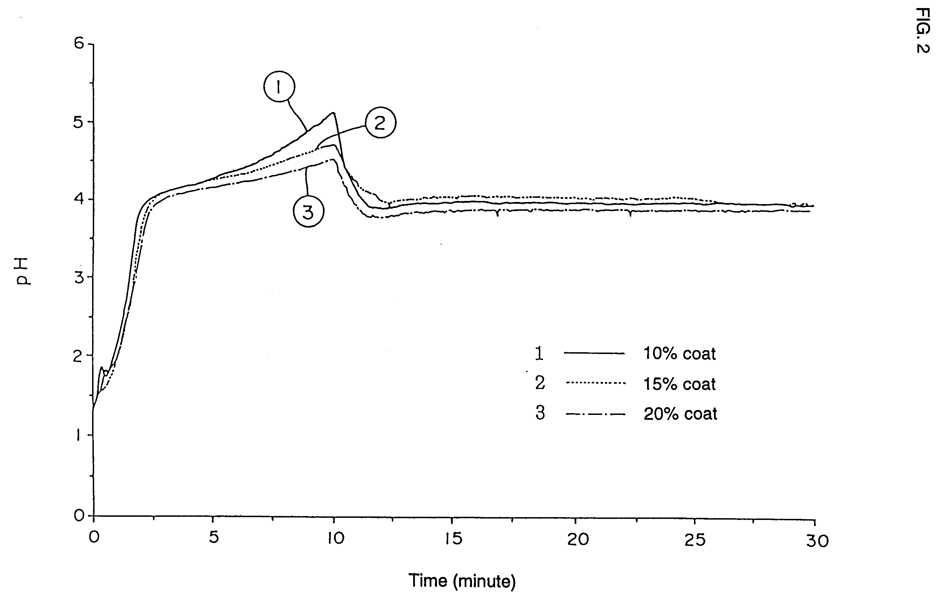

With respect to the coated product obtained in Example 1-(a) and the chewable tablet obtained in Example 1-(b), the sustainment test of antacid capacity was conducted in a manner similar to Test Example 1 except that 0.2N hydrochloric acid was added at a rate of 1 ml/min after 10 minutes, so that the test conditions would become severer (lower acid model) than those in Test Example 1. The coated product was weighed (400 g in terms of magnesium hydroxide), which was provided for the test together with 412 mg of dried aluminum hydroxide gel (225 mg in terms of aluminum oxide). The chewable tablet was provided for the test after crude pulverization. As is apparent from Fig. 2, the initial pH was high at the coating amount of 10%, while an increase in the initial pH was suppressed and the optimum pH could be controlled without being influenced by the long duration at the coating amount of 15% or greater. Even after tabletting, there is no substantial change in the pH profile and the optimum pH was maintained and sustained.

### Test Example 4

The coated products obtained in Example 1-(a), Example 2-(a), Example 3-(a) and Example 4-(a) were sieved, respectively. With respect to those having a particle size of 60-80 mesh, a pH controllability in 30 ml of 0.05N hydrochloric acid under stirring at 300 rpm and at 37°C were measured and suppressibility of an initial increase in pH was tested. The test was conducted without using dried aluminum hydroxide gel but using only the coated products. Incidentally, the test was conducted within a range (about 10 minutes) in which there is a fear of excessive increase in the initial pH showing. With respect to the sample which did not show an excessive increase in the initial pH, the measurement was continued until the increase was observed. As a result, it was found that an increase in the initial pH of any sample was suppressed.

**Table 2**

| | Example 1-(a) | Example 2-(a) | | Example 3-(a) | | Example 4-(a) | |
|---|---|---|---|---|---|---|---|
| | 20% coat | 40% coat | 60% coat | 40% coat | 60% coat | 40% coat | 60% coat |
| Initial pH | 1.31 | 1.27 | 1.32 | 1.29 | 1.35 | 1.32 | 1.29 |
| pH after 1 min. | 1.75 | 2.17 | 1.43 | 1.78 | 1.41 | 2.40 | 1.57 |
| pH after 2 min. | 2.37 | 4.19 | 1.56 | 2.35 | 1.47 | 5.26 | 1.95 |
| pH after 3 min. | 3.30 | 6.69 | 1.73 | 3.16 | 1.51 | 6.95 | 2.44 |
| pH after 4 min. | 4.82 | 8.41 | 1.91 | 4.09 | 1.57 | 8.53 | 3.22 |
| pH after 5 min. | 6.72 | 8.65 | 2.17 | 5.37 | 1.62 | 8.79 | 4.89 |
| pH after 6 min. | 8.38 | 8.75 | 2.54 | 6.99 | 1.68 | 8.88 | 6.90 |
| pH after 7 min. | 8.69 | 8.79 | 3.06 | 8.17 | 1.73 | 8.93 | 8.38 |
| pH after 8 min. | 8.83 | 8.80 | 3.89 | 8.49 | 1.79 | 8.97 | 8.68 |
| pH after 9 min. | 8.90 | 8.82 | 5.52 | 8.56 | 1.84 | 8.99 | 8.81 |
| pH after 10 min. | 8.95 | 8.83 | 7.44 | 8.63 | 1.90 | 8.98 | 8.88 |
| pH after 20 min. | / | / | / | / | 2.61 | / | / |
| pH after 30 min. | / | / | / | / | 3.62 | / | / |
| pH after 40 min. | / | / | / | / | 6.99 | / | / |

### Test Example 5

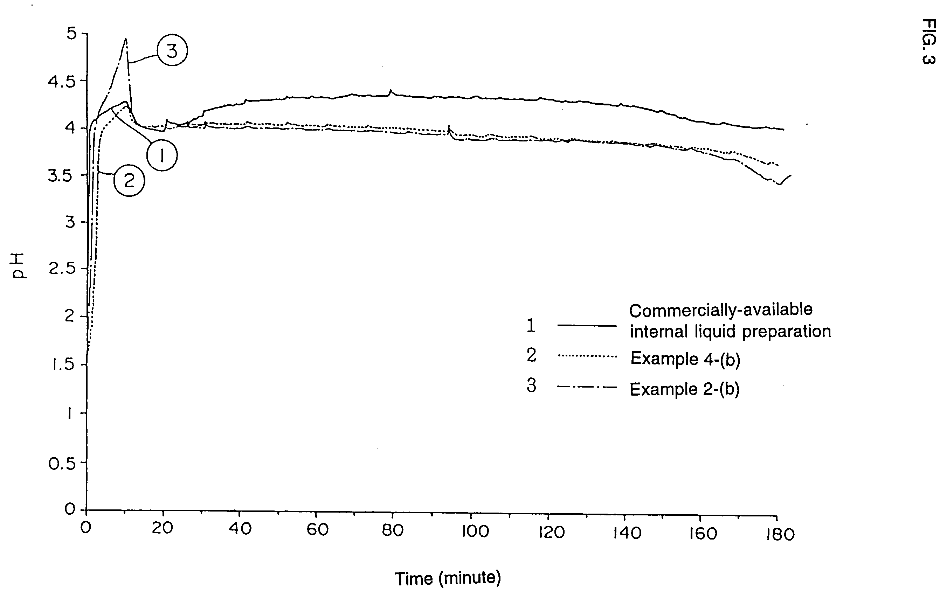

With respect to the tablets obtained in Example 2-(b) and Example 4-(b), the sustainment test of antacid capacity was conducted in a manner similar to Test Example 1. As is apparent from Fig. 3, increase in the initial pH of any tablet was suppressed and the optimum pH was sustained for a long period of term.

## Claims

1. A pharmaceutical composition comprising, as antacid ingredients of a solid antacid, a low neutralizing capacity antacid and a high neutralizing capacity antacid coated with a pH-independent and water-insoluble polymer base.

2. A pharmaceutical composition according to claim 1, which comprises, as the antacid ingredients of a solid antacid, a low neutralizing capacity antacid and a high neutralizing capacity antacid coated with a pH-independent and water-insoluble polymer base, said low neutralizing capacity antacid and said high-neutralizing capacity antacid showing the initial pH of less than 6 and the initial pH of 6 or higher, respectively, when tested in accordance with the modified Fuchs method using 30 ml of 0.05 N hydrochloric acid and a single dose.

3. A pharmaceutical composition according to claim 2, which comprises, as the antacid ingredients of a solid antacid, a low neutralizing capacity antacid and a high neutralizing capacity antacid coated with a pH-independent and water-insoluble polymer base; said low neutralizing capacity antacid and said high-neutralizing capacity antacid showing the initial pH of less than 6 and the initial pH of 6 or higher, respectively, when measured in accordance with modified Fuchs method using 30 ml of 0.05 N hydrochloric acid and a single dose; the amount of said low neutralizing capacity antacid *per se* and the amount of said high neutralizing capacity antacid *per se* are at a ratio of 0.2 - 2:1; and said high neutralizing capacity antacid being coated with a pH-independent and water-insoluble polymer bases in an amount of 5-100 weight % based on one antacid.

4. A pharmaceutical composition according to claim 3, wherein said pH-independent and water-insoluble polymer base is at least one polymer base selected from the group consisting of ethyl cellulose, aqueous dispersion-type ethyl cellulose, a Polyethylacrylate, methylmethacrylate, trimethylammonioethylmethacrylatechlorid and an emulsion of an ethyl acrylate-methyl methacrylate copolymer.

5. A pharmaceutical composition according to claim 3, wherein said high neutralizing capacity antacid is at least one antacid selected from the group consisting of sodium compound antacids, magnesium compound antacids and calcium compounds antacid.

6. A pharmaceutical composition according to claim 5, wherein said magnesium compound antacid is at least one antacid selected from the group consisting of magnesium oxide, magnesium hydroxide, magnesium carbonate and magnesium silicate.

7. A pharmaceutical composition according to claim 3, wherein the low neutralizing capacity antacid is an aluminum compound antacid.

8. A pharmaceutical composition according to claim 7, wherein the aluminum compound antacid is at least one antacid selected from the group consisting of dried aluminum hydroxide gel and aluminum silicate.

9. A pharmaceutical composition according to claim 8, wherein the low neutralizing antacid is dried aluminum hydroxide gel and the high neutralizing antacid is magnesium hydroxide.

10. A pharmaceutical composition according to claim 3, wherein the preparation form of the solid antacid is selected from the group consisting of chewable tablets, powders, granules, fine granules and capsules.

11. A process for the producing a pharmaceutical composition, which comprises spraying a solution or dispersion of a pH-independent and water-insoluble polymer base dissolved or dispersed in a solvent to a high neutralizing capacity antacid and then drying to effect coating, and then mixing the coated product, a low neutralizing capacity antacid and necessary ingredients for the preparation production to give a solid antacid.
